(19) Europäisches Patentamt
European Patent Office
Office européen des brevets

(11) Veröffentlichungsnummer: **0 268 192 B1**

(12) **EUROPÄISCHE PATENTSCHRIFT**

(45) Veröffentlichungstag der Patentschrift: **02.09.92**

(21) Anmeldenummer: **87116621.1**

(22) Anmeldetag: **11.11.87**

(51) Int. Cl.5: **C07C 69/54**, C07C 255/16, C07C 69/653, C07B 59/00, C07C 67/08, C08F 20/12

# (54) (Meth)Acrylsäureester.

(30) Priorität: **15.11.86 DE 3639117**

(43) Veröffentlichungstag der Anmeldung:
**25.05.88 Patentblatt 88/21**

(45) Bekanntmachung des Hinweises auf die Patenterteilung:
**02.09.92 Patentblatt 92/36**

(84) Benannte Vertragsstaaten:
**AT BE CH DE ES FR GB IT LI LU NL SE**

(56) Entgegenhaltungen:
**EP-A- 0 144 712**
**DE-A- 1 954 548**
**US-A- 3 681 438**
**US-A- 4 575 188**
**US-A- 4 645 856**

(73) Patentinhaber: **HOECHST AKTIENGESELLSCHAFT**
**Postfach 80 03 20**
**W-6230 Frankfurt am Main 80(DE)**

(72) Erfinder: **Wegener, Peter, Dr.**
**Am Eichkopf 4**
**W-6240 Königstein/Taunus(DE)**
Erfinder: **Heumüller, Rudolf, Dr.**
**Sodener Weg 1**
**W-6232 Bad Soden am Taunus(DE)**

**EP 0 268 192 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung bezieht sich auf Ester der ganz oder teilweise deuterierten (Meth)Acrylsäure mit Alkoholen, welche keinen oder nur wenig Wasserstoff im Molekül besitzen, Verfahren zur Herstellung dieser Ester und deren Verwendung zur Herstellung von transparenten polymeren Materialien für Lichtwellenleiter. Verschiedene Ester von perdeuterierter Methacrylsäure und ihre Anwendung zur Herstellung von transparenten Polymeren für Lichtwellenleiter sind bekannt.

So sind bekannt Fluoralkylester von deuterierter Methacrylsäure der Formel

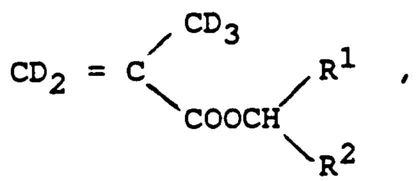

worin $R^1$ und $R^2$ niedere Fluoralkylreste oder Wasserstoffatome darstellen, wobei jedoch mindestens einer der beiden Reste ein niederer Fluoralkylrest sein soll (vgl. JP-OS 61-20906). Namentlich erwähnt sind 2,2,2-Trifluorethyl, 2,2,3,3-Tetrafluor-1-propyl und 1,1,1,3,3,3-Hexafluor-2-propyl. Allerdings enthalten diese Ester noch relativ viele Wasserstoffatome im Molekül, welche dämpfend auf die Lichtdurchlässigkeit der aus diesen Estern hergestellten Polymeren wirken.

Weiterhin sind Ester der ganz oder teilweise deuterierten Methacrylsäure mit Borneol, Isoborneol und Fenchylalkohol bekannt (vgl. EP-A 144712; US-PS 4575188). In der Alkoholkomponente dieser Ester tragen allerdings die Methylgruppen beträchtlich zum Restwasserstoffgehalt der Ester bei, da ihre Wasserstoffatome nur schwierig durch Deuterium ersetzt werden können.

Bekanntermaßen wird durch ein Wasserstoffatom pro Mol Monomer die Dämpfung der Lichtdurchlässigkeit des Polymers im Wellenlängenbereich von 600 bis 800 nm infolge von Absorption um ca. 11 dB/km angehoben. Es war nun wünschenswert, Monomere zu finden, die den daraus druch Polymerisation hergestellten polymeren transparenten Materialien eine möglichst geringe Dämpfung verleihen. Gleichzeitig sollten die transparenten polymeren Materialien einen Glaspunkt $T_G$ besitzen, welcher höher ist als der des bisher hauptsächlich verwendeten Polymethylmethacrylats ($T_G$ = 105 °C).

Es wurde gefunden, daß die Aufgabe gelöst werden kann, wenn ganz oder teilweise deuterierte (Meth)-Acrylsäure mit bestimmten Alkoholen, welche keinen oder nur wenig Wasserstoff im Molekül besitzen, verestert wird.

Somit betrifft die Erfindung eine Verbindung der Formel (I)

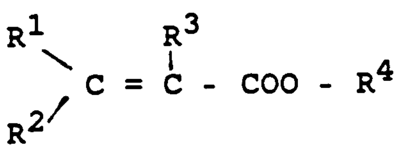 (I),

worin
$R^1$ und $R^2$ gleich oder verschieden sind und ein Wasserstoff-oder Deuteriumatom bedeuten,
$R^3$ H, D, $-CH_3$, $-CH_2D$, $-CHD_2$ oder $-CD_3$ ist und
$R^4$ eine der Gruppen $-C(CH_3)_2-CN$, $-C(CD_3)_2-CN$,

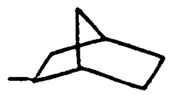 (Bicyclo-2.2.1-heptyl-2) oder

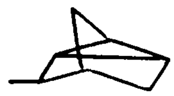 (Tricyclo-2.2.1$^{2,6}$-heptyl-3),

bedeutet, wobei die Ringe deuteriert sein können, und, wenn $R^3$ $-CD_3$ ist,
$R^4$ zusätzlich $-C(CF_3)_2-CF(CF_3)_2$, $-CF(CF_3)_2$ oder $-CD(CF_3)_2$ bedeutet.

Weiterhin betrifft die Erfindung ein Verfahren zu ihrer Herstellung und ihre Verwendung zur Herstellung transparenter polymerer Materialien.

In der Formel (I) sind $R^1$ und $R^2$ vorzugsweise ein Deuteriumatom, ist $R^3$ vorzugsweise ein Deuteriumatom oder eine der Gruppen $-CH_3$, $-CH_2D$, $-CHD_2$ oder $-CD_3$, insbesondere ein Deuteriumatom oder eine Perdeuteromethylgruppe, ist $R^4$ vorzugsweise

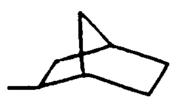 (Bicyclo-2.2.1-heptyl-2, Norbornyl),

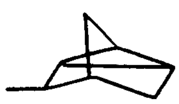 (Tricyclo-2.2.1.0$^{2.6}$-heptyl-3),

wobei die Ringe deuteriert sein können, und im Falle, daß $R^3 = -CD_3$ ist, zusätzlich

$-C(CF_3)_2-CF(CF_3)_2$, $-CF(CF_3)_2$ oder $-CD(CF_3)_2$.

Die Säurekomponente der erfindungsgemäßen Ester sind somit vorzugsweise deuterierte Acrylsäure und Methacrylsäure. Die Herstellung der deuterierten Säuren ist an sich bekannt, sie kann beispielsweise auf die folgende Art durchgeführt werden.

In einem Reaktor werden Methyl(meth)acrylat, Deuteriumoxid, ein Edelmetallsalz und ein Polymerisationsinhibitor mehrere Stunden bei einer Temperatur um 100 °C gerührt.

Auch ist es möglich, perdeuterierte Acrylsäure nach der folgenden Reaktionsgleichung zu gewinnen:

$$H\text{-}C\equiv C\text{-}COOR \xrightarrow[D_2O]{Ca(OH)_2} D\text{-}C\equiv C\text{-}COOR \xrightarrow[D_2\text{-Gas}]{\text{Lindlar-Katalysator}} D_2C{=}CD\text{-}COOR'$$

Aus den Estern lassen sich bei Bedarf die entsprechenden Säuren gewinnen.

Für die weitere Umsetzung (Veresterung) wird die Säure der Formel II

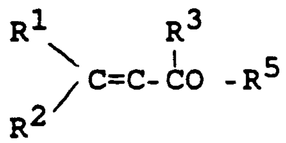

(II),

worin $R^1$, $R^2$ und $R^3$ die obengenannte Bedeutung haben, als solche ($R^5$ = OH oder OD) oder in Form eines Säurehalogenids ($R^5$ = Cl oder Br, vorzugsweise Cl) eingesetzt. Das Säurehalogenid wird mit Hilfe eines üblichen Halogenierungsmittels hergestellt, z.B. Oxalylchlorid, Phosphorpentachlorid, Phosphortrichlorid, Phosphorxychlorid, Benzoylchlorid, Benzotrichlorid, Phosphortribromid und insbesondere Thionylchlorid. Die Halogenierung mit Thionylchlorid erfolgt vorzugsweise in Gegenwart eines Katalysators wie Dimethylformamid. Die Umsetzung wird in einem aromatischen Kohlenwasserstoff, z.B. Toluol, Xylol oder Trimethylbenzol, als Lösungsmittel durchgeführt, und die Reaktionstemperatur liegt im Bereich von 50 bis 100 ° C vorzugsweise 70 bis 90 ° C.

Sodann wird die (Meth)Acrylsäure bzw. das Säurehalogenid mit einer Verbindung der Formel (III)

$HOR^4$ (III),

worin
$R^4$ die obengenannte Bedeutung hat, umgesetzt.

Für die Veresterung der Alkohole $HO-C(CH_3)-CN$, $HO-CD(CF_3)_2$, $HO-CF(CF_3)_2$ und $HO-C(CF_3)_2-CF-(CF_3)_2$ wird vorzugsweise das Säurehalogenid verwendet.

Die Veresterung wird vorzugsweise in einem Lösungsmittel durchgeführt, und die Reaktionstemperatur beträgt -10 bis 50 ° C, vorzugsweise 0 bis 25 ° C. Als Lösungsmittel dient ein polares organisches Lösungsmittel, insbesondere ein symmetrischer, asymmetrischer oder cyclischer Ether, z.B. Diethylether, Dipropylether, Diisopropylether, tert.-Butylmethylether, Tetrahydrofuran und Dioxan, ein aliphatischer Halogenkohlenwasserstoff, vorzugsweise Chlorkohlenwasserstoff, z.B. Dichlormethan, Trichlormethan, Tetrachlormethan, 1,1-Dichlorethan und 1,2-Dichlorethan, ein aromatischer Halogenkohlenwasserstoff, vorzugsweise Chlorkohlenwasserstoff, z.B. Chlorbenzol und 1,2- oder 1,3-Dichlorbenzol, oder ein aliphatisches oder aromatisches Nitril, z.B. Acetonitril und Benzonitril. Das Lösungsmittel kann auch ein Gemisch von mehreren polaren Lösungsmitteln sein. Es ist zweckmäßig, die Veresterung des Säurehalogenids mit dem Alkohol in Gegenwart einer organischen Base durchzuführen, insbesondere eines Trialkylamins mit jeweils 1 bis 4 Kohlenstoffatomen in den Alkylgruppen. Die Base wird in einer Menge von 0,5 bis 2 mol, vorzugsweise 0,8 bis 1,2 mol, eingesetzt (bezogen auf 1 mol Säurehalogenid). Aus dem Reaktionsgemisch wird der erhaltene Ester durch Destillation, vorzugsweise bei einem Druck von 1013 bis 200 mbar, oder - nach destillativer Entfernung des Lösungsmittels - durch Heißextraktion des festen Rückstandes mit einem unpolaren Lösungsmittel, vorzugsweise einem aliphatischen Kohlenwasserstoff wie n-Hexan, und anschließende Kristallisation isoliert. Es ist zweckmäßig, die Destillation in Gegenwart eines üblichen Polymerisationsinhibitors, z.B. Hydrochinon oder Hydrochinonmonomethylether, durchzuführen; dieser wird in einer Menge von 100 bis 500 ppm (bezogen auf Säurehalogenid verwendet. Die Sumpftemperatur liegt im Bereich von 20 bis 100 ° C, vorzugsweise 30 bis 85 ° C. Zur weiteren Reinigung wird der Ester erneut destilliert, vorzugsweise bei vermindertem Druck, oder umkristallisiert.

Der Alkohol wird in einer Menge von 0,5 mol, vorzugsweise 0,8 bis 1,2 mol, eingesetzt (bezogen auf 1 mol (Meth)Acrylsäure).

Eine weitere Veresterungsmethode ist das Arbeiten in Gegenwart eines wasserentziehenden Mittels, beispielsweise Oleum.

Die (Meth)Acrylsäureester des Bicycloheptyl-(Norbornyl-) und des Tricycloheptylalkohols können nach bekannten Methoden durch säurekatalysierte Addition der (Meth)Acrylsäure an Bicyclohepten bzw. Bicycloheptadien hergestellt werden.

Die erfindungsgemäßen Ester ergeben nach bekannter radikalischer Polymerisation allein, untereinander und/oder anderen Comonomeren, beispielsweise Methacrylsäureester aliphatischer oder alicyclischer Alkohole, transparente polymere Materialien, welche eine geringe Dämpfung für eingestrahltes Licht und eine hohe Glastemperatur $T_G$ besitzen. Die für die Herstellung der erfindungsgemäßen Ester verwendeten Alkohole enthalten entweder keinen oder nur wenig Wasserstoff im Molekül.

Die cyclischen Alkohole vom Norbornen-Typ (Bicyclo-2.2.1-heptanol-2 und Tricyclo-2.2.1.$0^{2.6}$-heptanol-3) bieten gegenüber Borneol und Isoborneol, deren Methacrylsäureester bekannt sind, den Vorteil eines günstigeren C/H-Verhältnisses und die Möglichkeit, auf Grund einer Wagner-Meerwein-Umlagerung alle

Wasserstoffatome durch katalytischen H/D-Austausch mit $D_2O$ durch Deuterium zu ersetzen. Da bei der Additionsreaktion ein Gemisch von exo- und endo-Norbornylalkohol-2 bzw. exo- und endo-Tricycloheptyalkohol-3 entsteht, wird die Anisotropie eines aus den Estern hergestellten Polymerisats verringert, wodurch die Transparenz erhöht wird.

Die aus den Estern erhaltenen transparenten polymeren Materialien werden zur Herstellung von Lichtwellenleitern, Resistmaterialien, Linsen, optischen Datenspeichermedien und anderen transparenten Gegenständen verwendet.

Die Erfindung wird durch die nachfolgenden Beispiele näher erläutert.

**Beispiel 1**

Methacrylsäure-2-cyan-isopropylester

Zu 10 ml Methacrylsäurechlorid (0,103 M) in 50 ml Methyl-tert.-Butylether wurden 9 ml 2-Cyan-isopropylalkohol (0,098 M) gegeben, danach unter Eiskühlung 15 ml Triethylamin zugetropft und der Ansatz 1 Stunde gerührt. Das Triethylammoniumhydrochlorid wurde abfiltriert, das Filtrat vom Lösungsmittel befreit und im Vakuum destilliert. Bei 49 - 50°C/1,3 mbar wurden 7,9 g Methacrylsäure-2-cyan-isopropylester abdestilliert, entsprechend 51,6 % Ausbeute. IR($CH_2Cl_2$): C=O bei 1750 $cm^{-1}$, C=N bei 2290 $cm^{-1}$ (schwach)
Molmasse: 153

**Beispiel 2**

Die per-deuterierte Verbindung wurde analog aus Methacrylsäurechlorid-D5 und 2-Cyan-isopropylalkohol-D6 hergestellt. Ausbeute 90 g, Siedepunkt: 53°C/1,3 mbar.
Molmasse : 164

**Beispiel 3**

In je 5 ml des Methacrylsäure-2-cyan-isopropylesters gemäß Beispiel 1 und 2 wurde jeweils 50 mg Di-Lauroylperoxid gelöst und die Lösungen 20 Stunden bei 50°C, dann 2 Stunden bei 90°C gehalten. Man erhielt glasklare Polymere, deren Glastemperatur bei 117°C liegt, Zersetzungstemperatur 220°C.

**Beispiel 4**

Methacrylsäure-norbornylester

165 g Norbornen (= 1,75 M) wurden in 100 ml Methylenchlorid gelöst, dazu wurde 1 g tert. Butylbrenzcatechin als Stabilisator gegeben. Unter Rühren und Kühlung wurde bei 30 - 40°C eine Mischung von 150 ml Methacrylsäure und 15 ml $BF_3$-Etherat zugetropft, Dauer 2 Stunden. Die Mischung wurde noch 2 Stunden gerührt, die Lösung mit Wasser neutral gewaschen, getrocknet und durch Destillation aufkonzentriert. Der dickflüssige Rückstand wurde bei Kp= 52°C/0,013 mbar im Ölpumpen-Vakuum destilliert. Man erhielt 210 g Methacrylsäure-norbornylester, entsprechend 66 % Ausbeute. IR-($CH_2Cl_2$) C=O bei 1710 $cm^{-1}$.
Molmasse: 180

Das $H^1$-NMR-Spektrum war im Einklang mit der angegebenen Struktur.

**Beispiel 5**

Ausgehend von Methacrylsäure-D5 und partiell deuterierten Norbonen konnte der (im Norbornyl-Rest partiell) deuterierte Ester analog Beispiel 4 hergestellt werden, wobei die Ausbeute und Siedepunkt der undeuterierten Verbindung entsprechen.

**Beispiel 6**

Je 5 ml der reinen Verbindungen aus Beispiel 4 und Beispiel 5 wurden mit je 50 mg Dilauroylperoxid 20 Stunden bei 50°C, dann 2 Stunden bei 90°C gehalten. Man erhielt glasklare, harte Polymere. Die Differentialthermoanalyse ergab 2 $T_G$-Werte von 120,8°C und 172°C, Zersetzungstemperatur 225°C.

**Beispiel 7**

Perdeutero-methacrylsäure-perfluor-dimethylbutyl-ester

10 ml Methacrylsäurechlorid-D5, gelöst in 50 ml Methyl-tert.-Butylether, wurde mit 40 g Perfluordimethyl-butan-2-ol versetzt. Unter Rühren und Kühlung wurden 17 ml Triethylamin eingetropft. Die Temperatur wurde bei 20 ° C gehalten. Nach 1 Stunde wurde das ausgefallene Triethylammoniumhydrochlorid abgesaugt und das Filtrat destilliert. Der Perdeuteromethacrylsäure-perfluordimethylbutylester siedete bei 45 ° C/11 mbar. Die Ausbeute betrug 14 g, entsprechend 28,5 % d.Th.

Der Ester war mit 1 % Dilauroylperoxid polymerisierbar (20 h bei 50 ° C, 1 h bei 90 ° C) und lieferte ein glasklares Polymerisat mit einer Glastemperatur von 111 ° C.

**Beispiel 8**

Acrylsäure-tricyclo-2.2.1.0$^{2.6}$-heptyl-3-ester

30 ml Norbornadien (= 0,32 M) wurden in 50 ml $CH_2Cl_2$ gelöst und zu der Lösung bei 20 ° C eine Mischung von 20 ml Acrylsäure (= 0,29 M) 0,5 g tert.-Butylbrenzcatechin und 3 ml $BF_3$-Etherat unter Rühren zugetropft.

Man beobachtete eine leicht exotherme Reaktion. Nach 6 Stunden wurde der Ansatz in einen Scheidetrichter überführt und dort mit Wasser gewaschen und danach getrocknet. Bei der folgenden Destillation gingen bei 60 ° C und 1,7 mbar 12,6 g einer Substanz über. Die Substanz hatte eine Molmasse von 164, aus dem $H^1$- und $C^{13}$-NMR-Spektrum ergab sich die Struktur eines Acrylsäure-tricyclo-2.2.1.0$^{2.6}$-heptyl-3-esters. Ausbeute 26,5 %.

**Beispiel 9**

Methacrylsäure-tricyclo-2.2.1.0$^{2.6}$-heptyl-3-ester

Analog Beispiel 8 wurden 35 ml Norbornadien, gelöst in 50 ml $CH_2Cl_2$, mit einer Mischung von 25 ml Methacrylsäure, 5 ml $BF_3$-Etherat und 1 g tert.-Butylbrenzcatechin (als Stabilisator) versetzt. Nach 6 Stunden leicht exothermer Reaktion wurde der Ansatz wie im Beispiel 8 aufgearbeitet.

Kp. 75 ° - 85 ° C bei 1,7 mbar, Ausbeute 32 g = 62 % d.Th.,
Molmasse 178.

Aus dem $H^1$- und $C^{13}$-NMR Spektrum ergab sich die Struktur eines Methacrylsäure-tricyclo-2.2.1.0$^{2.6}$-heptyl-3-esters.

**Beispiel 10**

Perdeutero-methacrylsäure-deuterohexafluorisopropylester

30 ml perdeuterierte Methacrylsäure und 45 ml perdeuteriertes Hexazfluorisopropanol (hergestellt durch katalytische Hydrierung von Hexafluoraceton mit $D_2$ an einem Pd/C-Katalysator) wurden mit 75 ml 16 %igem Oleum gemischt. Dabei stieg die Temperatur auf 40 ° C. Nach 15 Minuten wurde die Mischung in einen Dünnschichtverdampfer überführt und bei einer Wandtemperatur von 130 ° C und einem Vakuum von 44 mbar destilliert. Bei einer Kopftemperatur von 50 bis 60 ° C gingen 60 g roher Perdeuteromethacrylsäure-deuterohexafluorisopropylester über, welcher nochmals destilliert wurde. Man erhielt 50 g Endprodukt, entsprechend einer Ausbeute von 60 % d.Th.

Der Ester ließ sich mittels 0,1 Gew.-% Dilauroylperoxid polymerisieren und lieferte ein Polymerisat mit einem $T_G$-Wert von 74 ° C und einer Zersetzungstemperatur größer als 200 ° C.

**Beispiel 11**

Perdeutero-methacrylsäure-perfluorisopropylester

12,9 g 0,222 M) Kaliumflorid wurden in 80 ml Diglykoldimethylether suspendiert. In diese Suspension wurden bei Raumtemperatur 33 g (0,198 M) Hexafluoraceton eingeleitet. Dann wurde der Ansatz noch 2 Stunden gerührt und danach das nicht gelöste Kaliumfluorid abgetrennt. In das Filtrat wurden bei

Raumtemperatur 22 g (0,2 M) Methacrylsäurechlorid-$D_5$ eingetropft und das Reaktionsgemisch noch 1 Stunde weitergerührt. Nach Abtrennung des entstandenen Feststoffes wurde das Filtrat destilliert.

Farblose Flüssigkeit, KP. 45,2 - 45,4 °C/80 mbar. Ausbeute 32 g = 64 % d.Th.

Der Ester ließ sich mittels 0,5 Gew.-% Dilauroylperoxid während 24 Stunden bei 50 °C zu einem glasklaren Polymeren mit einem $T_G$-Wert von 76 °C polymerisieren.

## Patentansprüche

**Patentansprüche für folgende Vertragsstaaten : AT, BE, CH, DE, FR, GB, IT, LI, LU, NL, SE**

1. Verbindung der Formel (I)

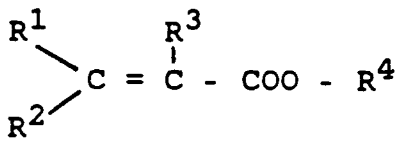

(I),

worin
$R^1$ und $R^2$ gleich oder verschieden sind und ein Wasserstoff-oder Deuteriumatom bedeuten,
$R^3$ H, D, $-CH_3$, $-CH_2D$, $-CHD_2$ oder $-CD_3$ ist und
$R^4$ eine der Gruppen $-C(CH_3)_2-CN$, $-C(CD_3)_2-CN$,

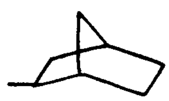

(Bicyclo-2.2.1-heptyl-2) oder

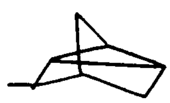

(Tricyclo-2.2.1$^{2,6}$-heptyl-3),

bedeutet, wobei die Ringe deuteriert sein können, und, wenn $R^3$ $-CD_3$ ist, $R^4$ zusätzlich $-C(CF_3)_2-CF(CH_3)_2$, $CF(CF_3)_2$ oder $-CD(CF_3)_2$ bedeutet.

2. Verbindung nach Anspruch 1, dadurch gekennzeichnet, daß in Formel (I)
$R^3$ -D, $-CH_3$, $-CH_2D$, $-CHD_2$ oder $-CD_3$ ist und
$R^4$

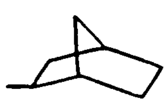

(Bicyclo-2.2.1-heptyl-2)

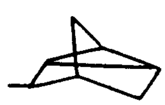

(Tricyclo-2.2.1.0$^{2.6}$-heptyl-3),

wobei die Ringe deuteriert sein können, und im Falle von $R^3$ = $-CD_3$ zusätzlich $-C(CF_3)_2-CF(CF_3)_2$, $CF(CF_3)_2$ oder $-CD(CF_3)_2$ bedeutet.

3. Verfahren zur Herstellung der Verbindung gemäß Anspruch 1 durch Umsetzung einer Verbindung der Formel (II)

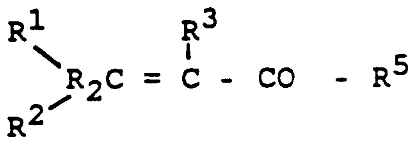 (II),

worin $R^1$,$R^2$ und $R^3$ die in Anspruch 1 genannte Bedeutung haben und $R^5$ ein Halogenatom, eine OH-Gruppe oder eine OD-Gruppe ist, mit einer Verbindung der Formel (III)

$HOR^4$ (III),

worin $R^4$ die in Anspruch 1 genannte Bedeutung hat, bei einer Temperatur von -10 bis 50 °C in Gegenwart eines Katalysators.

**4.** Verwendung der Verbindung gemäß Anspruch 1 zur Herstellung transparenter polymerer Materialien.

**Patentansprüche für folgenden Vertragsstaat: ES**

**1.** Verfahren zur Herstellung der Verbindung der Formel (I)

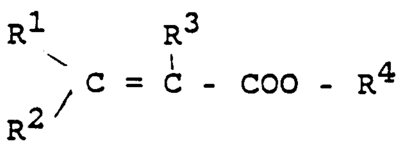 (I),

worin
$R^1$ und $R^2$ gleich oder verschieden sind und ein Wasserstoff-oder Deuteriumatom bedeuten,
$R^3$ H, D, $-CH_3$, $-CH_2D$, $-CHD_2$ oder $-CD_3$ ist und
$R^4$ eine der Gruppen $-C(CH_3)_2-CN$, $-C(CD_3)_2-CN$,

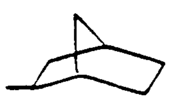 (Bicyclo-2.2.1-heptyl-2) oder

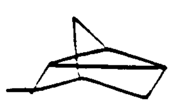 (Tricyclo-2.2.1$^{2,6}$-heptyl-3),

bedeutet, wobei die Ringe deuteriert sein können, und, wenn $R^3$ $-CD_3$ ist,
$R^4$ zusätzlich $-C(CF_3)_2-CF(CH_3)_2$, $CF(CF_3)_2$ oder $-CD(CF_3)_2$ bedeutet,
durch Umsetzung einer Verbindung der Formel (II)

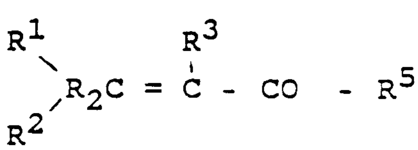 (II),

worin $R^1$,$R^2$ und $R^3$ die in Anspruch 1 genannte Bedeutung haben und $R^5$ ein Halogenatom, eine OH-Gruppe oder eine OD-Gruppe ist, mit einer Verbindung der Formel (III)

$HOR^4$ (III),

worin $R^4$ die in Anspruch 1 genannte Bedeutung hat, bei einer Temperatur von -10 bis 50°C in Gegenwart eines Katalysators.

**2.** Verwendung der gemäß Anspruch 1 hergestellten Verbindung zur Herstellung transparenter polymerer Materialien.

**Claims**

**Claims for the following Contracting States : AT, BE, CH, DE, FR, GB, IT, LI, LU, NL, SE**

**1.** A compound of the formula (I)

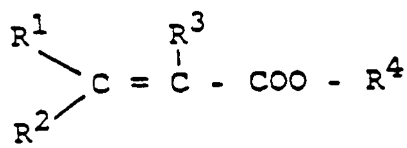

(I)

wherein
$R^1$ and $R^2$ are identical or different and are a hydrogen atom or deuterium atom,
$R^3$ is H, D, $-CH_3$, $-CH_2D$, $-CHD_2$ or $-CD_3$ and
$R^4$ is one of the groups $-C(CH_3)_2-CN$, $-C(CD_3)_2-CN$,

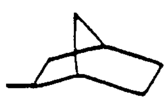

(bicyclo-2.2.1-hept-2-yl) or

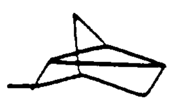

(tricyclo-2.2.$1^{2.6}$-hept-3-yl),

it being possible for the rings to be deuterated, and, if $R^3$ is $-CD_3$,
$R^4$ can also be $-C(CF_3)_2-CF(CF_3)_2$, $-CF(CF_3)_2$ or $-CD(CF_3)_2$.

**2.** A compound as claimed in claim 1, wherein the formula (I),
$R^3$ is -D, $-CH_3$, $-CH_2D$, $-CHD_2$ or $-CD_3$ and
$R^4$ is

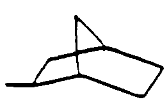

(bicyclo-2.2.1-hept-2-yl)

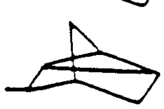

(tricyclo-2.2.1.$0^{2.6}$-hept-3-yl),

it being possible for the rings to be deuterated, and, in the case of $R^3$ = $-CD_3$, can also be $-C(CF_3)_2-CF(CF_3)_2$, $-CF(CF_3)_2$ or $-CD(CF_3)_2$.

**3.** A process for preparing the compound as claimed in claim 1 by reacting a compound of the formula (II)

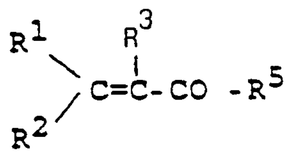

(II)

wherein $R^1$, $R^2$ and $R^3$ are as defined in claim 1 and $R^5$ is a halogen atom, an OH group or an OD group, with a compound of the formula (III)

$HOR^4$ (III)

wherein $R^4$ is as defined in claim 1, at a temperature from -10 to 50 °C in the presence of a catalyst.

**4.** The use of the compound as claimed in claim 1 for the production of transparent polymeric materials.

**Claims for the following Contracting State : ES**

**1.** A process for preparing the compound of the formula (I)

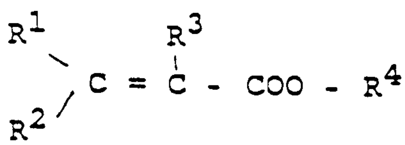

(I)

wherein
$R^1$ and $R^2$ are identical or different and are a hydrogen atom or deuterium atom,
$R^3$ is H, D, $-CH_3$, $-CH_2D$, $-CHD_2$ or $-CD_3$ and
$R^4$ is one of the groups $-C(CH_3)_2-CN$, $-C(CD_3)_2-CN$,

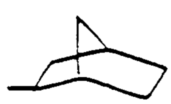

(bicyclo-2.2.1-hept-2-yl) or

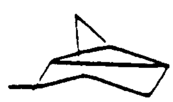

(tricyclo-2.2.$1^{2.6}$-hept-3-yl),

it being possible for the rings to be deuterated, and, if $R^3$ is $-CD_3$,
$R^4$ can also be $-C(CF_3)_2-CF(CF_3)_2$, $-CF(CF_3)_2$ or $-CD(CF_3)_2$ by reacting a compound of the formula (II)

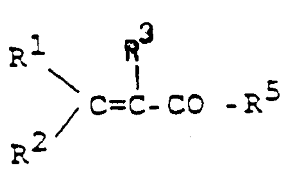

(II)

wherein $R^1$, $R^2$ and $R^3$ are as defined in claim 1 and $R^5$ is a halogen atom, an OH group or an OD group, with a compound of the formula (III)

$HOR^4$ (III)

wherein $R^4$ is as defined in claim 1, at a temperature from -10 to 50 °C in the presence of a catalyst.

**2.** The use of the compound prepared as claimed in claim 1 for the production of transparent polymeric materials.

**Revendications**
**Revendications pour les Etats contractants suivants : AT, BE, CH, DE, FR, GB, IT, LI, LU, NL, SE**

**1.** Les composés de formule I ci-dessous :

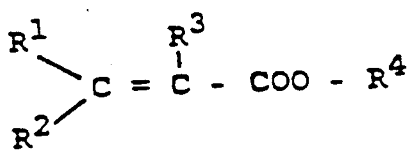 (I),

dans laquelle :
$R^1$ et $R^2$, qui peuvent être identiques ou différents l'un de l'autre, représentent chacun un atome d'hydrogène ou de deutérium,
$R^3$ représente H, D, $-CH_3$, $-CH_2D$, $-CHD_2$ ou $-CD_3$ et
$R^4$ l'un des groupes $-C(CH_3)_2-CN$, $-C(CD_3)_2-CN$,

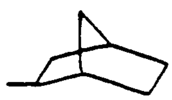 (Bicyclo-2.2.1-heptyle-2) ou

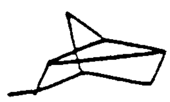 (Tricyclo-2.2.1$^{2,6}$-heptyle-3),

les cycles pouvant être deutériés, et, si $R^3$ est un groupe $-CD_3$,
$R^4$ peut être également un groupe $-C(CF_3)_2-CF(CF_3)_2$, $-CF(CF_3)_2$ ou $-CD(CF_3)_2$.

**2.** Les composés selon la revendication 1, caractérisés en ce que dans la formule I :
$R^3$ est -D, $-CH_3$, $-CH_2D$, $-CHD_2$ ou $-CD_3$ et
$R^4$ le groupe

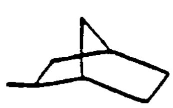 (Bicyclo-2.2.1-heptyle-2, norbornyle) ou

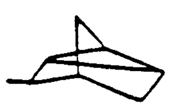 (Tricyclo-2.2.1$^{2,6}$-heptyle-3),

les cycles pouvant être deutériés, et dans le cas où $R^3$ est le groupe $-CD_3$, $R^4$ peut être en outre le groupe $-C(CF_3)_2-CF(CF_3)_2$,$-CF(CF_3)_2$ ou $-CD(CF_3)_2$.

**3.** Préparation des composés selon la revendication 1 par réaction d'un composé de formule II :

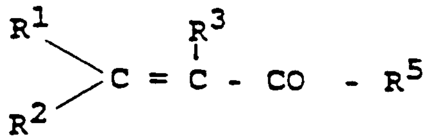 (II),

$R^1$, $R^2$, $R^3$ ayants la signification donnée pour la formule (I).
$R^5$ désignant un atome d'halogène ou bien un groupe OH ou OD, avec un composé de formule III

$HOR^4$ (III),

dans laquelle $R^4$ ayant la signification donnée pour la formule (I,), à une température de -10 à 50 °C en présence d'un catalyseur.

**4.** L'emploi des composés de la revendication 1 pour en faire des matières polymères transparentes.

**Revendications pour l'Etat contractant suivant : ES**

**1.** Procédé de préparation des composés de formule I :

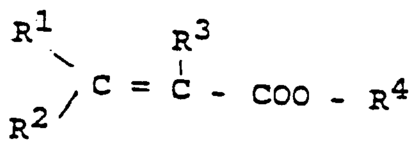 (I)

dans laquelle :
$R^1$ et $R^2$, qui peuvent être identiques ou différents l'un de l'autre, représentent chacun un atome d'hydrogène ou de deutérium,
$R^3$ représente H, D, $-CH_3$, $-CH_2D$, $-CHD_2$ ou $-CD_3$ et
$R^4$ l'un des groupes $-C(CH_3)_2-CN$, $-C(CD_3)_2-CN$,

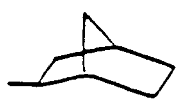 (Bicyclo-2.2.1-heptyle-2) ou

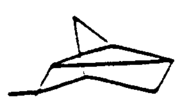 (Tricyclo-2.2.1$^{2,6}$-heptyle-3),

les cycles pouvant être deutériés, et, si $R^3$ est un groupe $-CD_3$,
$R^4$ peut être également un groupe $-C(CF_3)_2-CF(CF_3)_2$, $-CF(CF_3)_2$ ou $-CD(CF_3)_2$ par réaction d'un composé de formule II :

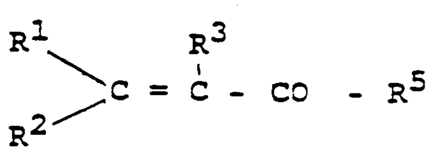 (II)

$R^1$, $R^2$, $R^3$ ayants la signification donnée pour la formule (I).
$R^5$ désignant un atome d'halogène ou bien un groupe OH ou OD, avec un composé de formule III.

$HOR^4$ (III)

dans laquelle $R^4$ ayant la signification donnée pour la formule (I), à une température de -10 à 50°C en présence d'un catalyseur.

**2.** L'emploi des composés obtenus selon la revendication 1 pour en faire des matières polymères transparentes.